# EUROPEAN PATENT SPECIFICATION

(11) **EP 2 747 760 B1**
(45) Date of publication and mention of the grant of the patent: **15.07.2015**
(21) Application number: 12725163.5
(22) Date of filing: 10.05.2012
(51) Int. Cl.: A61K 31/19, A61K 47/36

(54) **STERILE ALGINATE-BASED AQUEOUS COMPOSITION FOR MEDICAL USE AND PROCESS FOR THE PREPARATION THEREOF**
STERILE ALIGNATBASIERTE WÄSSRIGE ZUSAMMENSETZUNG ZUR MEDIZINISCHEN VERWENDUNG UND VERFAHREN ZUR HERSTELLUNG DAVON
COMPOSITION AQUEUSE STÉRILE À BASE D'ALGINATE DESTINÉE À UN USAGE MÉDICAL ET SON PROCÉDÉ DE PRÉPARATION

(30) Priority: 15.09.2011 EP 11181482
(43) Date of publication of application: 02.07.2014
(73) Proprietor: Bender Analytical Holding B.V., 6611 KH Overasselt (NL)
(72) Inventor: Bender, Johannes Caspar Mathias Elizabeth, 6611 KH Overasselt (NL); Pellikaan, Hubert Clemens, NL-3572 CA Utrecht (NL)
(74) Representative: Nederlandsch Octrooibureau
(86) International application number: PCT/NL2012/050318
(87) International publication number: WO 2013/039380

(56) References cited:
- WO-A2-2009/032158
- BECKER TIMOTHY A; KIPKE DARYL R; BRANDON TEDD: "Calcium alginate gel: A biocompatible and mechanically stable polymer for endovascular embolization", JOURNAL OF BIOMEDICAL MATERIALS RESEARCH, vol. 54, no. 1, 2001, pages 76-86, XP002678675,

## Description

### TECHNICAL FIELD OF THE INVENTION

The present invention relates to sterile alginate-based aqueous compositions for medical uses. The alginate-based aqueous compositions of the present invention can advantageously be used, for instance, to prevent adhesions between a healing trauma site and adjacent surrounding tissue. These compositions can further be used in implants or in pharmaceutical preparations for oral administration.

The invention further relates to a process for the preparation of such aqueous alginate-based compositions.

### BACKGROUND OF THE INVENTION

Adhesions are unwanted tissue growths occurring between layers of adjacent bodily tissue or between tissues and internal organs. Adhesions are often formed during the dynamic process of healing of the incision and tissue trauma after surgery. The initiation of the adhesion begins with the formation of a fibrin matrix. The ischemic conditions caused by surgery prevent fibrinolytic activity to dissolve the matrix, and the fibrin persists. Wound repair cells then turn the matrix into an organized adhesion, often having a vascular supply and neuronal elements. Adhesions can prevent the normal motions of tissues and organs with respect to their neighbouring structures. Adhesions are a particular problem in gastrointestinal and gynaecological surgery, leading to post-operative bowel obstruction, infertility, and chronic pelvic pain.

The medical and scientific communities have studied ways of reducing the formation of post-surgical adhesions by the use of high molecular weight carboxyl-containing biopolymers. These biopolymers can form hydrated gels which act as physical barriers to separate tissues from each other during healing, so that adhesions between normally adjacent structures do not form. After healing is substantially complete, the barrier is no longer needed, and should be eliminated from the body to permit more normal function of the affected tissues.

KR 2001 107 067 describes an adhesion preventing agent comprising 1.0-15 wt% of alginate having a viscosity of 150 centipoise, 0-2.5wt% of polyethylene glycol having a molecular weight of 3,000-5,000, 0-8 wt% of agarose, 0-1 wt.% of an antibiotic, and 73.5-99.0 wt% of water. The adhesion preventing agent is sterilized at a temperature of less than 150°C for 5-20 minutes.

US 5,693,624 (Johnson & Johnson Medical Inc.) describes an aqueous gel composition for use as a wound dressing comprising from 2% to 10% w/v of a water-soluble alginate salt and from 1% to 40% w/v of a C₃-C₆ dihydric or trihydric alcohol, said composition being substantially sterile and having been sterilized by heat sterilization. In the US patent it is stated that the inclusion of relatively large amount of polyhydric alcohol (more than 15% w/v) results in an alginate gel that is stabilised against hydrolysis and consequent loss of viscosity during autoclave sterilization.

US 6,150,581 (United States Surgical Corporation) describes a method for preventing post surgical adhesions comprising:
- providing an aqueous solution of chitosan and a complexing agent;
- providing an aqueous solution of alginate; and
- combining the chitosan/complexing agent solution with the alginate solution to form an anti-adhesion barrier at a site of surgical intervention.

US 6,638,917 (Boston Scientific SciMed, Inc) describe a method of forming a sheet for use as an adhesion barrier, comprising:
- forming a film from an alginate solution; and
- contacting the film with a cross-linking solution to form a cross-linked mechanically stable sheet for placement of at least a portion of the sheet at a site of trauma to create the adhesion barrier

WO 2006/044342 A2 (FMC Biopolymer AS) describes a method of using a self gelling alginate dispersion to prevent post surgical adhesion formation in an individual, said method comprising dispensing a self gelling alginate dispersion within an individual by:
a) forming a dispersion by mixing i) a solution comprising a soluble alginate with an insoluble alginate/gelling ion particles or ii) immediately soluble alginate, insoluble alginate/gelling ion particles and a solvent, and
b) dispensing the dispersion whereby the dispersion forms an alginate gel matrix.

The present invention aims to provide a sterile, ready-to-use alginate-based aqueous composition of neutral pH that has excellent storage stability, that can be directly applied as such at the site of trauma, and that is easy to manufacture. In particular, the present invention relates to a sterile alginate-based aqueous gel formulation that can be produced using heat sterilization of the total composition and that is heat stable as well as storage stable in terms of pH and rheological properties (e.g. viscosity).

### SUMMARY OF THE INVENTION

The inventors have discovered that the aforementioned desirable features can be realized in an alginate-based aqueous composition that has been sterilized by heat sterilization and that contains 0.5-10 wt.% of an alginate salt and 10-500 mM of one or more dissolved C₈-C₁₂ fatty acid salts, said alginate-based composition further being characterized by a pH of 6.5-7.5 and a viscosity at 25 °C of at least 300 cP.

The specifications for alginate-based compositions have to be very tight given that these compositions are often applied in critically ill patients and furthermore, because they are applied directly at the site of surgical trauma. However, meeting such tight specifications poses a major challenge in case the composition is alginate-based.

It is known from e.g. Holme et al. 2008 (Kinetics and mechanisms of depolymerization of alginate and chitosan in aqueous solution. Carbohydrate Polymers 2008, Vol. 73, 656-664), Home et al. 2003 (Thermal depolymerization of alginate in the solid state. Carbohydrate Polymers 2003, Vol. 54, 431-438.) and Bradley et al. (The Determination of Kinetics of Polysaccharide Thermal Degradation using High Temperature Viscosity Measurements. Carbohydrate Polymers 1988, Vol. 9, 257-267) that both heat sterilization and storage induce decomposition of alginate, resulting in time-dependent changes of pH and viscosity.

The inventors have also found that heat sterilization, especially moist heat sterilization above 100 °C, tends to adversely effect both the pH-stability and rheological stability of alginate-based aqueous compositions. Investigations undertaken by the inventors ultimately showed that this particular instability was somehow linked to unsuitable buffering systems (e.g. phosphate buffers) that are used to render these compositions biocompatible. In addition, the inventors have unexpectedly discovered that alginate-based aqueous compositions of neutral pH containing 0.01-0.5 M of one or more dissolved C₈-C₁₂ fatty acid salts do not suffer from this instability and that alginate-based compositions that are buffered by these fatty acid salts can suitably be heat sterilized. Examples of C₈-C₁₂ fatty acid salts that may suitably be employed include caprylate, pelargonate, caprate, undecylate, laurate and combinations thereof.

The present invention also provides a process for preparing an alginate-based aqueous composition as described above, said process comprising combining a water-soluble alginate salt; one or more fatty acid components selected from C₈-C₁₂ fatty acids and salts thereof; and water, followed by heat sterilization

### DETAILED DESCRIPTION OF THE INVENTION

Accordingly, one aspect of the invention relates to an aqueous alginate-based composition that has been sterilized by heat sterilization and having a viscosity at 25 °C of at least 300 cP (Helipath® T F spindle, 100 rpm), said composition having a pH in the range of 6.5-7.5; containing 0.5-10 wt.% of an alginate salt; and further containing 10-500 mM of one or more dissolved C₈-C₁₂ fatty acid salts.

Whenever reference is made in this document to a "Cₙ fatty acid", what is meant is an unbranched, saturated or unsaturated monocarboxylate substance that contains n carbon atoms, including the carbon atom of the carboxyl group.

Although the inventors do not wish to be bound by theory, it is believed that the C₈-C₁₂ fatty acid salts act as buffering agents. Here the term "buffering agent" refers to substances that can be used in aqueous systems to drive an acidic or basic solution to a certain pH (e.g. a pH within the range of 6.5-7.5) and that prevent a change in this pH, for instance, as a result of heat sterilisation or storage.

Buffering agents can be either the weak acid or weak base that would comprise a buffer solution (an aqueous solution comprising a mixture of a weak acid and its conjugate base or a weak base and its conjugate acid). Buffering agents are the substances that are responsible for the buffering seen in buffer solutions. Buffering agents are similar to buffer solutions in that buffering agents are the main components of buffer solutions. They both regulate the pH of a solution and resist changes in pH. A buffer solution maintains the pH for the whole system which is placed into it, whereas a buffering agent can be added to an already acidic or basic solution, which it then modifies and maintains a new pH.

Typically, the alginate-based composition has a viscosity at 25 °C of not more than 10,000 cP. Preferably, said viscosity lies within the range of 500-8,000 cP, even more preferably of 700-4,000cP. The alginate-based composition advantageously has a sufficiently high viscosity to prevent it from flowing away from the trauma site and a sufficiently low viscosity to allow it to spread out over the trauma site.

The alginate-based composition of the present invention offers the advantage that it is ready-for-use and that it is a single component system, as opposed to some of the two-component systems described in the prior art. The present composition can be a viscous liquid or a gel that can be rendered flowable by applying pressure or shear (e.g. a thixotropic gel).

Alginates are hydrophilic biopolymers with the unique ability to form heat-stable gels that can develop and set at physiologically relevant temperatures. Alginates are a family of non-branched binary copolymers of 1-4 glycosidically linked β-D-mannuronic acid (M) and α-L-guluronic acid (G) residues. The relative amount of the two uronic acid monomers and their sequential arrangement along the polymer chain vary widely, depending on the origin of the alginate. Alginate is the structural polymer in marine brown algae such as *Laminaria hyperborea, Macro cystis pyrifera, Lessonia nigrescens* and *Ascophyllum nodosum*. Alginate is also produced by certain bacteria such as *Pseudomonas aeruginosa*, *Azotobacter vinelandii* and *Pseudomonas fluoresceins* (WO 04/011628).

The present invention utilizes an alginate salt, preferably an alginate metal salt. Even more preferably, the alginate salt comprises one or more cations selected from Na⁺, K⁺, Ca²⁺ and Mg²⁺.

The amount of alginate salt contained in the present alginate-based composition preferably lies within the range of 1-5 wt.%. Most preferably, the alginate salt content of the composition is in the range of 1.2-4 wt.%.

Best results are obtained with the present alginate-based composition if the a high molecular weight alginate salt is employed. Accordingly, the alginate salt advantageously has a molecular weight of at least 50,000 g/mol, even more preferably of at least 400,000 g/mol.

The present aqueous composition can advantageously be used as an anti-adhesion composition. Alternative medical applications include the use as an implant or as an oral dosage unit. Especially in case of the latter applications, the aqueous composition advantageously contains a pharmaceutically active ingredient. It will be understood that for the aforementioned medical applications it is highly desirable to employ an ultrapure alginate, i.e. an alginate from which endotoxins have been removed almost completely. Preferably, the alginate employed in accordance with the present invention is an alginate that meets the standard laid down in ASTM F2064-00 (reapproved 2006).

It is well-known that alginate gels can be produced when a multivalent cation (e.g. Ca²⁺) forms ionic bonds with the negatively charged group from a G residue from two or more different alginate polymers, thereby cross-linking these polymers. The formation of multiple cross-linkages among numerous alginate polymers results in the matrix that is the alginate gel structure.

According to a preferred embodiment at least a part of the alginates salt contained in the alginate-based composition is cross-linked by divalent cations selected from Ca²⁺, Mg²⁺ and combinations thereof. Advantageously, the alginate in the present composition is only moderately cross-linked in order to prevent the composition from becoming a rigid gel. Typically, the total content of divalent cations selected from Ca²⁺, Mg²⁺ and combinations thereof is within the range of 10-3000 µmol per gram of alginate, more preferably 20-1200 µmol per gram of alginate per gram of alginate and most preferably 40-800 µmol per gram of alginate per gram of alginate. Moderate cross-linking of the alginate was found to reduce the pH drift observed during sterilisation. Furthermore, the inventors have observed that such cross-linking improves stability of pH and viscosity during storage.

The total content of monovalent cations selected from Na⁺, K⁺ and combinations thereof preferably lies within the range of 0.5-50 mmol, more preferably 1-30 mmol and most preferably 2-20 mmol per gram of alginate

The amount of the one or more dissolved C₈-C₁₂ fatty acid salts employed in the present alginate-based composition preferably lies within the range of 5 to 500 mmol/l. Even more preferably, the amount of this buffering agent is within the range of 10 to 100 mmol/l.

The one or more C₈-C₁₂ fatty acid salts employed in accordance with the present invention may be saturated or unsaturated. Preferably, these fatty acid salts are salts of saturated fatty acids.

In accordance with a preferred embodiment of the present invention, the one or more dissolved C₈-C₁₂ fatty acid salts are one or more dissolved C₈-C₁₁ fatty acid salts, more preferably C₈-C₁₀ fatty acid salts, even more preferably C₈-C₉ fatty acid salts. Most preferably, the fatty acid salts employed are caprylic acid salts.

The fatty acid salts contained in the present composition preferably are alkali metal salt. Accordingly, it is preferred that the composition contains 5 -500 mM of alkali metal cations selected from Na⁺, K⁺ and combinations thereof. Typically, the alkali metal cations and the one or more C₈-C₁₂ fatty acids are contained in the composition in a molar ratio that lies within the range of 0.5:1 to 1:1, more preferably in the range of 0.8:1 to 1:1.

According to a preferred embodiment, the present composition additionally contains 5-500 mM, more preferably 10-100 mM of one or more saturated or unsaturated, linear C₂-C₄ mono- or dicarboxylates that are optionally substituted with up to 2 hydroxyl groups. Examples of these mono- and dicarboxylates include acetate, propionate, crotonate, succinate, fumarate and tartarate. Most preferably, the present composition contains 10-500 mM of acetate.

In accordance with another preferred embodiment, the alginate composition of the present invention contains 0.01-50 mM, more preferably 0.1-10 mM of carbonate.

A particularly advantageous embodiment of the present invention is composition that comprises the one or more dissolved C₈-C₁₂ fatty acid salts, the one or more C₂-C₄ mono- or dicarboxylates and carbonate. The inventors have discovered that the use of this particular combination of buffering agents offers the advantage that the pH drop that is normally observed during heat sterilization of the composition can be minimized effectively.

As explained herein before, the alginate-based composition of the present invention offers the important advantage that its pH remains stable during storage, even when the product is stored at elevated temperatures. Accordingly, in accordance with another preferred embodiment, the pH of the composition remains within the range of 6.5-7.5 when the composition is kept at 40 °C for 6 months.

In order to ensure that, for instance, atmospheric carbon dioxide will not influence the pH of the alginate-based composition, said composition is preferably packaged without a headspace or with a headspace that contains no carbon dioxide. Even more preferably, the headspace contains an inert gas such as nitrogen.

The alginate-based composition of the present invention is surprisingly stable under sterilization conditions, notably moist heat sterilization at temperatures well above 100 °C. This characteristic is evident from the fact that the composition can be heated to high temperature for a significant amount of time without resulting in a substantial viscosity decrease and/or pH change. Thus, a preferred alginate-based composition meets the condition that its viscosity at 25 °C of the aqueous composition does not drop by more than 50% if the aqueous composition is heated to 121 °C for 15 minutes.

The bulk of the present alginate-based aqueous composition consists of water. Typically, the alginate-based composition contains 95-99 wt.% of water.

Besides the alginate salt, the one or more C₈-C₁₂ fatty acid salts and the water, the alginate-based composition may suitably contain other components, such a pharmaceutically active substances (e.g. antimicrobials, anti-inflammatories), dextran sulphate, dermatan sulphate, pentosan polysulphate, sodium chloride etc.

To limit the alginate's potential tendency to promote the growth of intra-abdominal anaerobic bacteria, possibly leading to intra-abdominal abscess formation or causing localized peritonitis to develop into generalized peritonitis, the alginate based composition advantageously contains a biocompatible anti-microbial agent, to inhibit growth of intra-abdominal anaerobic bacteria.

The aqueous alginate-based composition of the present invention, unlike the sterile wound dressing compositions described in US 5,693,624, preferably contains less than 15% (w/v) of polyhydric alcohol, e.g. a C₃-C₆ polyhydric alcohol such as propylene glycol or hexylene glycol. Even more preferably the alginate-based composition contains less than 10% (w/v), most preferably less than 1% (w/v) polyhydric alcohol.

Likewise, the alginate-based composition of the present invention, unlike the adhesion preventing agent taught by KR 2001 107 067 does not contain polyethylene glycol having a molecular weight of 3,000-5,000.

The aqueous alginate-based composition preferably is more or less isotonic. Thus, the alginate-based composition preferably contains Cl⁻ in a concentration of 20-300 mmol/l, more preferably in a concentration of 50-250 mmol/l.

Another aspect of the invention relates to a process for preparing an alginate-based aqueous composition as defined herein before, said process comprising combining a water-soluble alginate salt; one or more fatty acid components selected from C₈-C₁₂ fatty acids and salts thereof; and water, followed by moist heat sterilisation.

Preferably, the sterilization conditions employed in the present process achieve a sterility assurance level of less than 10⁻⁵, more preferably of less than 10⁻⁶.

In the present process heat sterilisation is suitably achieved by heating to a temperature of at least 100 °C for at least 5 minutes. Even more preferably, said heat sterilization involves heating to a temperature of at least 110 °C for at least 5 minutes, more preferably for at least 10 minutes. Most preferably, the heat sterilization involves heating to a temperature of at least 121 °C for at least 5 minutes, especially at least 15 minutes.

According to a particularly preferred embodiment, the moist heat sterilization employs high pressure steam. Even more preferably, the moist heat sterilization is carried out in an autoclave.

As explained herein before, preferably at least some of the alginate salt is cross-linked by multivalent (metal) cations. Advantageously this cross-linking is achieved by combining an aqueous solution of the alginate salt with an aqueous solution containing multivalent cations. More particularly, the present process advantageously comprises combining an aqueous solution of 6-120 g/l of the water-soluble alginate salt with an aqueous solution containing 3 -250 mmol/l of divalent cations selected from Ca²⁺, Mg²⁺ and combinations thereof. According to a particularly preferred embodiment, the aqueous solution of the water-soluble alginate salt and the aqueous solution of the divalent cations are combined in a weight ratio that lies within the range of 1:2 to 10:1. Preferably, the one or more C₈-C₁₂ fatty acid salts are contained in the aqueous solution of the water-soluble alginate salt.

The present invention offers the advantage that pH of the alginate-based composition not only remains stable after heat sterilization, but also that the pH hardly changes during sterilization. Typically, the pH change observed during sterilization is less than 1.0 pH-unit, more preferably less than 0.5 pH-unit. Generally speaking, during sterilization the pH of the alginate-based composition remains within the range of 6.5-7.5.

The inventors have found that C₈-C₁₈ dicarboxylic acids may be used to replace the C₈-C₁₂ fatty acid salts in the present alginate composition. The results obtained with these dicarboxylic acids, however, are inferior to the results obtained with the C₈-C₁₂ fatty acid salts. The invention is further illustrated by means of the following non-limiting examples.

### EXAMPLES

### Example 1

Alginate-based aqueous compositions (100 ml) were prepared on the basis of the following recipe:

| **Ingredient** | **Amount** |
|---|---|
| Sodium alginate ^{#} | 2200 mg |
| CaCl₂ | 1.6 mg |
| Dextran sulphate | 53.8 mg |
| NaCl | 806 mg |
| Buffering agent | *Specified below* |
| Water | Remainder (to 100 ml) |

| | |
|---|---|
| ^{#} Manugel® DMB, high G alginate (visc. 300 cP; 1% aq. solution), ex FMC BioPolymer | |

The alginate-based aqueous compositions were compounded with three different physiologically acceptable buffers to produce a buffered composition having a pH of 7.0 :
- Composition 1: Octanoic acid (60 mM)
- Composition 2: Crotonic acid (0.1 M) + octanoic acid (60 mM)
- Composition 3: Succinic acid (0.1 M) + octanoic acid (60 mM)

In addition, a reference composition containing no buffering agent was prepared.

In all cases pH before sterilisation was adjusted to 7.0 with the help of 0.1N NaOH or 0.1N HCl.

Buffer compositions were made by accurately weighing the ingredients into the mixing flask and making up to volume with water for injection. Sodium chloride and dextran sulfate were then added to the buffer solution and dissolved. Ultrapure sodium alginate was then mixed into the solution to afford a homogeneous clear gel. A second solution containing the chosen buffer and calcium chloride was made after which it was combined with the gel.

Subsequently, the alginate-based aqueous compositions were sterilized by steam sterilisation (45 minutes at 121 °C). This sterilisation process is a worst case challenge. Normal sterilisation conditions achieve an SAL (sterility assurance level) of 10⁻⁶ by heating at 121 °C for 15 minutes.

Before and after sterilization, the viscosity of the different buffered alginate-based aqueous compositions at 25 °C was determined at 50 rpm, using a Helipath® T F spindle. Also the viscosity of the reference composition was determined, both before and after sterilization. Likewise, the pH of the buffered compositions and the reference composition was measured before and after sterilization.

The data so obtained are depicted in the following table

| | **Before sterilization** | | **After sterilization** | | **Viscosity change** | **pH change** |
|---|---|---|---|---|---|---|
| | **Viscosity (cP)** | **pH** | **Viscosity (cP)** | **pH** | | |
| 1 | 7,964 | 7.07 | 5,435 | 7.00 | -32% | -0.07 |
| 2 | 8,163 | 6.85 | 5,646 | 6.84 | -31% | -0.01 |
| 3 | 7,826 | 6.83 | 5,159 | 6.21 | -34% | -0.62 |
| Ref. | 7,926 | 6.40 | 2,683 | 6.49 | -66% | 0.09 |

These results show that the pH decrease in combination with the viscosity decrease observed after sterilization can be minimized by inclusion of a buffering agent containing a fatty acid salt.

Repeating experiments revealed that the pH of the non-buffered reference solution after sterilisation is unpredictable, even if the same experimental conditions are used. This is illustrated, for instance, by the results in the following table:

| **Composition** | **pH (before sterilization)** | **pH after sterilization*** |
|---|---|---|
| Ref. | 7.50 | 5.74 |

| | | |
|---|---|---|
| * sterilized by steam sterilization for 30 minutes at 121 °C. | | |

### Example 2

Example 1 was repeated except that this time standard sterilization conditions were employed (121 °C for 15 minutes). The effect of sterilization on viscosity (25 °C; 50 rpm) and pH is shown in the following table.

| | **Before sterilization** | | **After sterilization** | | **Viscosity change** | **pH change** |
|---|---|---|---|---|---|---|
| | **Viscosity (cP)** | **pH** | **Viscosity (cP)** | **pH** | | |
| 1 | 7,964 | 7.07 | 6,763 | 7.12 | -15% | 0.05 |
| 2 | 8,163 | 6.85 | 7,030 | 6.90 | -14% | 0.05 |
| 3 | 7,826 | 6.83 | 6,653 | 6.75 | -15% | -0.08 |
| Ref. | 7,926 | 6.40 | 4,256 | 6.90 | -46% | 0.50 |

These results show that the pH decrease in combination with the viscosity decrease, observed after sterilization, can be minimized by inclusion of a buffering agent containing a fatty acid salt.

### Comparative example 1

Example 1 was repeated, except that this time a phosphate buffer (28 mM) was used. Furthermore, the pH was adjusted to 7.5 before sterilization.

The phosphate buffer used comprised 67 parts by weight of NaH₂PO₄ and 220 parts by weight of Na₂HPO₄.

The data obtained (viscosity measured at 25 °C; 50 rpm) were as follows:

| | **Before sterilization** | | **After sterilization** | |
|---|---|---|---|---|
| **Buffering agent** | **Viscosity (cP)** | **pH** | **Viscosity (cP)** | **pH** |
| Phosphate | 6,777 | 7.5 | 216 | 6.9 |

Although the comparative example with phosphate buffer is also within a pH 6.5 - 7.5 range after sterilization, the drop in pH of 0.6 is one of the highest of all experiments and the decrease in viscosity is dramatically high.

### Comparative example 2

Comparative example 1 was repeated, except that this time the alginate-based aqueous composition was sterilized by steam sterilisation for 30 minutes at 121 °C and the viscosity was determined at 25 °C and 100 rpm.

The data obtained were as follows:

| | **Before sterilization** | | **After sterilization** | |
|---|---|---|---|---|
| **Buffering agent** | **Viscosity** | **pH** | **Viscosity** | **pH** |
| Phosphate | 600 | 7.5 | <50 | 6.05 |

This experiment shows that the pH of the phosphate buffered solution after sterilisation is unpredictable and that the viscosity decrease is very high.

In this respect it is noted that changes in pH or viscosity observed during sterilization are highly indicative of the stability (pH and viscosity) during prolonged storage. In other words, compositions showing little change in pH and viscosity during sterilization exhibit good storage stability. However, compositions that show substantial changes in pH or viscosity during sterilization exhibit poor storage stability.

## Claims

1. An aqueous composition for medical use that has been sterilized by heat sterilization and having a viscosity at 25 °C of at least 300 cP (Helipath® T F spindle, 100 rpm at 25 °C), said composition having a pH in the range of 6.5-7.5; containing 0.5-10 wt.% of an alginate salt; and further containing 10-500 mM of one or more dissolved C₈-C₁₂ fatty acid salts.

2. Aqueous composition according to claim 1, wherein the aqueous composition has a viscosity at 25 °C of less than 10,000 cP, preferably of 500-8,000 cP.

3. Aqueous composition according to any one of the preceding claims, wherein the aqueous composition contains 1-5 wt.% of the alginate salt.

4. Aqueous composition according to any one of the preceding claims, wherein the alginate salt has a molecular weight of at least 50,000 g/mol, more preferably at least 400,000 g/mol

5. Aqueous composition according to any one of the preceding claims, wherein the alginate salt is cross-linked by divalent cations selected from Ca²⁺, Mg²⁺ and combinations thereof.

6. Aqueous composition according to any one of the preceding claims, wherein the total content of divalent cations selected from Ca²⁺, Mg²⁺ and combinations thereof is within the range of 10-3000 µmol per gram of alginate, more preferably 20-1200 µmol per gram of alginate.

7. Aqueous composition according to any one of the preceding claims, wherein the one or more dissolved C₈-C₁₂ fatty acid salts are salts of saturated fatty acids.

8. Aqueous composition according to any one of the preceding claims, wherein the one or more dissolved C₈-C₁₂ fatty acid salts are one or more dissolved C₈-C₁₀ fatty acid salts.

9. Aqueous composition according to claim 8, wherein the one or more dissolved C₈-C₁₂ fatty acid salts are caprylic acid salts.

10. Aqueous composition according to any one of the preceding claims, wherein the composition contains 10-500 mM of one or more saturated or unsaturated, linear C₂-C₄ mono- or dicarboxylates that are optionally substituted with up to 2 hydroxyl groups.

11. Aqueous composition according to any one of the preceding claims, wherein the composition additionally contains 0.01-10 mM carbonate.

12. A process for preparing an aqueous composition according to any one of the preceding claims, said process comprising combining a water-soluble alginate salt; one or more fatty acid components selected from C₈-C₁₂ fatty acids and salts thereof; and water, followed by heat sterilisation.

13. Process according to claim 12, wherein prior to the heat sterilisation the process comprises combining an aqueous solution of 6-120 g/l of the water-soluble alginate salt with an aqueous solution containing 3 -250 mmol/l of divalent cations selected from Ca²⁺, Mg²⁺ and combinations thereof.

14. Process according to claim 13, wherein the aqueous solution of the water-soluble alginate salt and the aqueous solution of the divalent cations are combined in a weight ratio that lies within the range of 1:2 to 10:1.

15. Process according to any one of claims 12-14, wherein the heat sterilisation comprises heating the composition to a temperature of at least 100 °C for at least 5 minutes.

## Patentansprüche

1. Wässrige Zusammensetzung zur medizinischen Verwendung, die durch Wärmesterilisation sterilisiert worden ist und bei 25 °C eine Viskosität von mindestens 300 cP (Helipath^{®} -TF-Spindel, 100 U/min bei 25 °C) aufweist,
wobei die Zusammensetzung einen pH-Wert im Bereich von 6,5 - 7,5 aufweist; 0,5 - 10 Gew.-% eines Alginatsalzes enthält, und des Weiteren 10 - 500 mM eines oder mehrerer gelöster C₈-C₁₂-Fettsäuresalze enthält.

2. Wässrige Zusammensetzung nach Anspruch 1, wobei die wässrige Zusammensetzung bei 25 °C eine Viskosität von kleiner 10.000 cP, vorzugsweise von 500 - 8.000 cP aufweist.

3. Wässrige Zusammensetzung nach einem der vorhergehenden Ansprüche, wobei die wässrige Zusammensetzung 1 - 5 Gew.-% des Alginatsalzes enthält.

4. Wässrige Zusammensetzung nach einem der vorhergehenden Ansprüche, wobei das Alginatsalz ein Molekulargewicht von mindestens 50.000 g/mol, bevorzugter mindestens 400.000 g/mol aufweist.

5. Wässrige Zusammensetzung nach einem der vorhergehenden Ansprüche, wobei das Alginatsalz durch zweiwertige Kationen quervernetzt ist, ausgewählt aus Ca²⁺, Mg²⁺ und Kombinationen davon.

6. Wässrige Zusammensetzung nach einem der vorhergehenden Ansprüche, wobei der Gesamtgehalt an zweiwertigen Kationen, ausgewählt aus Ca²⁺, Mg²⁺ und Kombinationen davon, in dem Bereich von 10 - 3000 µmol pro Gramm Alginat, bevorzugter 20 - 1200 µmol pro Gramm Alginat liegt.

7. Wässrige Zusammensetzung nach einem der vorhergehenden Ansprüche, wobei das eine oder die mehreren gelösten C₈-C₁₂-Fettsäuresalze Salze gesättigter Fettsäuren sind.

8. Wässrige Zusammensetzung nach einem der vorhergehenden Ansprüche, wobei das eine oder die mehreren gelösten C₈-C₁₂-Fettsäuresalze ein oder mehrere gelöste C₈-C₁₀-Fettsäuresalze sind.

9. Wässrige Zusammensetzung nach Anspruch 8, wobei das eine oder die mehreren gelösten C₈-C₁₂-Fettsäuresalze Caprylsäuresalze sind.

10. Wässrige Zusammensetzung nach einem der vorhergehenden Ansprüche, wobei die Zusammensetzung 10 bis 500 mM von einem oder mehreren gesättigten oder ungesättigten, linearen C₂-C₄-Mono- oder -Dicarboxylaten enthält, die wahlweise mit bis zu 2 Hydroxylgruppen substituiert sind.

11. Wässrige Zusammensetzung nach einem der vorhergehenden Ansprüche, wobei die Zusammensetzung zusätzlich 0,01 - 10 mM Carbonat enthält.

12. Verfahren zur Herstellung einer wässrigen Zusammensetzung nach einem der vorhergehenden Ansprüche, wobei das Verfahren das Kombinieren eines wasserlöslichen Alginatsalzes, eines oder mehrerer Fettsäurebestandteile, ausgewählt aus C₈-C₁₂-Fettsäuren und Salzen davon, und Wasser, gefolgt von einer Wärmesterilisation, umfasst.

13. Verfahren nach Anspruch 12, wobei das Verfahren vor der Wärmesterilisation das Kombinieren einer wässrigen Lösung von 6 - 120 g/l des wasserlöslichen Alginatsalzes mit einer wässrigen Lösung umfasst, die 3 - 250 mmol/l zweiwertige Kationen enthält, ausgewählt aus Ca²⁺, Mg²⁺ und Kombinationen davon.

14. Verfahren nach Anspruch 13, wobei die wässrige Lösung des wasserlöslichen Alginatsalzes und die wässrige Lösung der zweiwertigen Kationen in einem Gewichtsverhältnis kombiniert werden, das in einem Bereich von 1:2 bis 10:1 liegt.

15. Verfahren nach einem der Ansprüche 12 - 14, wobei die Wärmesterilisation das Erwärmen der Zusammensetzung auf eine Temperatur von mindestens 100 °C für mindestens 5 Minuten umfasst.

## Revendications

1. Une composition aqueuse pour un usage médical qui a été stérilisée par stérilisation thermique et qui présente une viscosité à 25°C d'au moins 300 cP (par broche TF Helipath®, 100 tours par minute à 25°C), ladite composition présentant un pH dans la plage de 6,5 à 7,5; contenant de 0,5 à 10% en poids de sel alginate et contenant en outre 10 à 500 mM d'un ou plusieurs sels d'acides gras en C₈-C₁₂ dissous.

2. Composition aqueuse selon la revendication 1, dans laquelle la composition aqueuse présente une viscosité à 25°C inférieure à 10 000 cP, de préférence de 500-8,000 cP.

3. Composition aqueuse selon l'une quelconque des revendications précédentes, dans laquelle la composition aqueuse contient de 1 à 5% en poids de sel alginate.

4. Composition aqueuse selon l'une quelconque des revendications précédentes, dans laquelle le sel alginate présente un poids moléculaire d'au moins 50 000 g/mol, plus préférablement d'au moins 400 000 g/mol.

5. Composition aqueuse selon l'une quelconque des revendications précédentes, dans laquelle le sel alginate est réticulé par des cations divalents choisis parmi Ca²⁺, Mg²⁺ et leurs combinaisons.

6. Composition aqueuse selon l'une quelconque des revendications précédentes, dans laquelle la teneur totale en cations divalents choisis parmi Ca²⁺, Mg²⁺ et leurs combinaisons est dans la plage de 10 à 3000 µmol par gramme d'alginate, de préférence de 20 à 1200 µmol par gramme d'alginate.

7. Composition aqueuse selon l'une quelconque des revendications précédentes, dans laquelle les un ou plusieurs sels d'acides gras en C₈ -C₁₂ dissous sont des sels d'acides gras saturés.

8. Composition aqueuse selon l'une quelconque des revendications précédentes, dans laquelle les un ou plusieurs sels d'acides gras en C₈-C₁₂ dissous sont un ou plusieurs sels d'acides gras en C₈ -C₁₀ dissous.

9. Composition aqueuse selon la revendication 8, dans laquelle les un ou plusieurs sels d'acides gras en C₈ -C₁₂ dissous sont des sels de l'acide caprylique.

10. Composition aqueuse selon l'une quelconque des revendications précédentes, dans laquelle la composition contient de 10 à 500 mM d'un ou plusieurs mono- ou dicarboxylates en C₂-C₄ linéaires, saturés ou insaturés, qui sont éventuellement substitués par au plus deux groupes hydroxyles.

11. Composition aqueuse selon l'une quelconque des revendications précédentes, dans laquelle la composition contient en outre de 0,01 à 10 mM de carbonate.

12. Procédé de préparation d'une composition aqueuse selon l'une quelconque des revendications précédentes, ledit procédé comprenant la combinaison d'un alginate soluble dans l'eau, d'un ou plusieurs composants d'acide gras choisis parmi les acides gras en C₈ - C₁₂ et leurs sels, et d'eau ; suivie d'une stérilisation thermique.

13. Procédé selon la revendication 12, dans lequel avant stérilisation thermique, le procédé comprend la combinaison d'une solution aqueuse de 6 à 120g/l de sel alginate soluble dans l'eau avec une solution aqueuse contenant 3 à 250 mmol/l de cations divalents choisis parmi Ca ²⁺, Mg ²⁺ et leurs combinaisons.

14. Procédé selon la revendication 13, dans lequel la solution aqueuse de sel alginate soluble dans l'eau et la solution aqueuse des cations divalents sont combinés dans un rapport en poids qui se situe dans la plage de 1/2 à 10/1.

15. Procédé selon l'une quelconque des revendications 12 à 14, dans lequel la stérilisation thermique comprend le chauffage de la composition à une température d'au moins 100°C pendant au moins 5 minutes.
